# EUROPEAN PATENT APPLICATION

(11) **EP 4 427 797 A1**
(43) Date of publication of application: **11.09.2024**
(21) Application number: 22889315.2
(22) Date of filing: 02.11.2022
(51) Int. Cl.: A61N 1/30

(54) **ELECTROCHEMICAL PUMP FOR MEDICAMENT DELIVERY, AND MEDICAMENT DELIVERY DEVICE THEREOF**

(30) Priority: 02.11.2021 CN 202111289447
(71) Applicant: Micromed Co., Ltd., New Taipei City, Taiwan (TW)
(72) Inventor: LI, Po-Ying, Taipei City, Taiwan (TW); CHENG, Tsung-Chieh, Kaohsiung City, Taiwan (TW)
(74) Representative: Straus, Alexander
(86) International application number: PCT/CN2022/129246
(87) International publication number: WO 2023/078293

(57) **Abstract**

The disclosure provides an electrochemical pump for drug delivery, and a drug delivery device thereof. The electrochemical pump includes: a substrate having a first surface, a second surface and a plurality of through holes, wherein the second surface has a plurality of contacts, and the through holes have a conductive material therein which is electrically connected to the plurality of contacts; a plurality of electrodes disposed on the first surface and electrically connected to the plurality of contacts on the second surface through the conductive material in the plurality of through holes; an electronic device disposed on the second surface and electrically connected to the contacts on the second surface; and a space for accommodating electrolyte, which has an electrolyte therein, and is adjacent to the first surface such that the plurality of electrodes is in contact with the electrolyte. The disclosure also provides a drug delivery device implementing the electrochemical pump.

## Description

### FIELD OF THE INVENTION

The disclosure relates to an electrochemical pump and a drug delivery device thereof, for use in automatic drug delivery.

### BACKGROUND OF THE INVENTION

Recently in the pharmaceutical industry, electrochemical pump technology for delivering therapeutic agents is considered an area for further development and improvement. However, the design of electrochemical pumps conventionally used to deliver therapeutic agents has defects which require improvement. For example, the electrolyte of the electrochemical pump using a microelectrode structure has high impedance and low local electric field which cause high power loss, as well as low solid-state free energy on the electrode surface. The aforementioned results in a problem of high power consumption of the overall pump system.

In addition, the electrodes and contact pads of conventional electrochemical pumps are usually located on the same side of the substrate, which result in that the connection structure materials of the control circuit board and the electrodes are inevitably corroded by the electrochemical electrolyte during the electrochemical reaction, seriously affecting the operation and energy consumption performances of the overall electrochemical pump system.

The actuation mechanism of electrochemical drug delivery medical devices is mainly by generating gas pressure as a source of driving power, for example, utilizing pressure difference between the interior of the drug delivery device and the environment around the device to deliver the drug. However, precise control of amplitude of the pressure generated in the device requires advanced device structural design and electronic control device design, and improper design thereof may make it difficult to accurately control the amount of gas generated for the purpose of accurately controlling a small amount of drug and stabilizing the drug delivery speed.

Further, when the drug delivery device is required to drive high-viscosity drugs in ultra-high speed, the power output by the electrochemical device needs to be increased accordingly so that it has the ability to output ultra-large driving force. Therefore, the circuit board and power supply must also be capable of outputting ultra-high output energy. For the same-side structural design between the contact pads and the electrodes, the lead sections between the contact pads and the electrodes greatly increase the overall resistance impedance, and in turn seriously affect the energy loss transmitted from the power supply to the electrodes, thus significantly reducing pressure power output performance of the overall electrochemical pump system.

In addition, when a large volume (such as several milliliters) of highly concentrated drug needs to be delivered by subcutaneous injection or intramuscular injection, it is necessary to adopt a gentler injection speed and a slightly longer injection time to avoid the problem of patient pain caused by injecting too fast. If injection delivery is performed manually, it not only costs labor, but also difficult to accurately control the rate of drug delivery to avoid the above-mentioned problem of pain. Currently, automatic delivery devices on the market mostly use mechanical force (such as springs) or micromotors as the source of driving power. However, unstable driving force caused by springs or micromotors may cause unexpected pain during the injection, and it is difficult to achieve both fast and stable delivery speed.

Therefore, it is urgently needed to improve and solve the above problems in the field, and develop an electrochemical pump structure that could avoid corrosion due to the electrodes and the lead pad located on the same side, as well as automatic drug delivery devices or methods with reduced impedance to increase the efficiency of energy transmission, increased control accuracy of energy transmission to reduce pain, and a small size.

### SUMMARY OF THE INVENTION

To achieve the objectives of the present invention, provided herein are an electrochemical pump for drug delivery and a drug delivery device thereof. The electrochemical pump comprises: a substrate having a first surface, a second surface and a plurality of through holes, wherein the second surface has a plurality of contacts, and the through holes have a conductive material therein which is electrically connected to the plurality of contacts; a plurality of electrodes disposed on the first surface and electrically connected to the plurality of contacts on the second surface through the conductive material in the plurality of through holes; an electronic device disposed on the second surface and electrically connected to the contacts on the second surface; and a space for accommodating electrolyte, which has an electrolyte therein, and is adjacent to the first surface such that the plurality of electrodes is in contact with the electrolyte.

In one embodiment, the electrode at least includes an anode and a cathode. The electrode may further include a reference electrode. The electrode may further include a redundant electrode.

In one embodiment, any of the electrodes is electrically connected to a plurality of contacts through the plurality of through holes.

In one embodiment, the first surface is coated with a hydrophilic layer.

In one embodiment, the space for accommodating electrolyte is formed by attaching a superabsorbent material to the first surface.

In one embodiment, the superabsorbent material is a sponge.

In one embodiment, the superabsorbent material is made of a material selected from the group consisting of: polyvinyl alcohol (PVA), polyethylene oxide (PEO), polyacrylic acid, poly lactic acid (PLA), polyglycolic acid (PGA), PLA/PGA copolymer, polycaprolactone (PCL), and a polymer fiber product thereof.

In one embodiment, the space for accommodating electrolyte is formed by attaching a breathable film to the first surface, wherein the space for accommodating electrolyte is sealed with the breathable film.

In one embodiment, the electronic device includes: a circuit board; an input port; and an outlet.

In one embodiment, the circuit board is electrically connected to the electrochemical pump substrate by the outlet, an end of the input port is electrically connected to the circuit board, and the other end is an input port of power supply.

In one embodiment, the electronic device further includes a driving circuit component.

In one embodiment, the electronic device includes a power supply.

In one embodiment, the circuit board is electrically connected to the contact by the outlet, the power supply is electrically connected to the circuit board via the input port, and the driving circuit component is electrically connected to the circuit board and is electrically connected to the power supply.

In one embodiment, the electronic device is detachable.

In another aspect, provided herein is a drug delivery device, comprising: a container for accommodating a drug, having opposing first opening and second openings, wherein the first opening is an outlet of the drug; a spacing member disposed in the container between the two openings, and capable of sliding freely along a wall of the container; and the electrochemical pump disposed at the second opening, wherein the electrochemical pump is sealably attached to the container to seal the second opening.

In one embodiment, the container is a syringe, the syringe can further include a needle disposed at the first opening.

In one embodiment, the container is transparent or translucent.

In one embodiment, the container is rigid or flexible.

In one embodiment, the spacing member is composed of a rubber plug.

### BRIEF DESCRIPTION OF THE DRAWINGS

Fig. 1 is a schematic structural diagram of an electrochemical pump disclosed herein.
Fig. 2 is a cross-sectional view of a drug delivery device disclosed herein.
Fig. 3 is a diagram showing the relation between electrochemical pump flow rate and number of through holes.
Fig. 4 is a diagram showing total electrochemical pump delivery volume versus number of through holes.

Reference numbers in the drawings: 10 electrochemical pump; 101 substrate; 1011 first surface; 1012 second surface; 1013 through hole; 1014 conductive material; 1015 contact; 102 space; 1021 electrolyte; 103 electrode; 104 electronic device; 1041 circuit board; 1042 input port; 1043 outlet; 1044 power supply; 200 drug delivery device; 20 syringe; 201 first chamber; 202 second chamber; 21 first opening; 22 second opening; 23 spacing member; 24 injection needle.

### DESCRIPTION OF THE INVENTION

All technical and scientific terms used in the present specification and claims, unless otherwise defined, have the definitions that are known to those skilled in the technical field to which the present invention belongs. The singular forms "a," "an," "the" and the like include plural referents unless the context clearly dictates otherwise. As used herein, "or" or "and" means "and/or" unless stated otherwise. In addition, the terms "comprising" and "including" are non-limiting open-ended transitional terms.. The foregoing definitions only illustrate the reference of the definitions of terms and should not be construed as limitations of the present invention. Unless otherwise stated, the materials used in the present invention are commercially available and easily available.

The ordinals recited in the specification and the claims such as "first," "second," etc., are intended only to describe the elements claimed and imply or represent neither that the claimed elements have any proceeding ordinals, nor that sequence between one claimed element and another claimed element or between steps of a manufacturing method. The use of these ordinals is merely to differentiate one claimed element having a certain designation from another claimed element having the same designation.

Further, the terms "on," "above," "over," or the like as used herein refer to not only direct contact with the other element (e.g., a substrate), but also indirect contact with the other element (e.g., a substrate).

### Examples

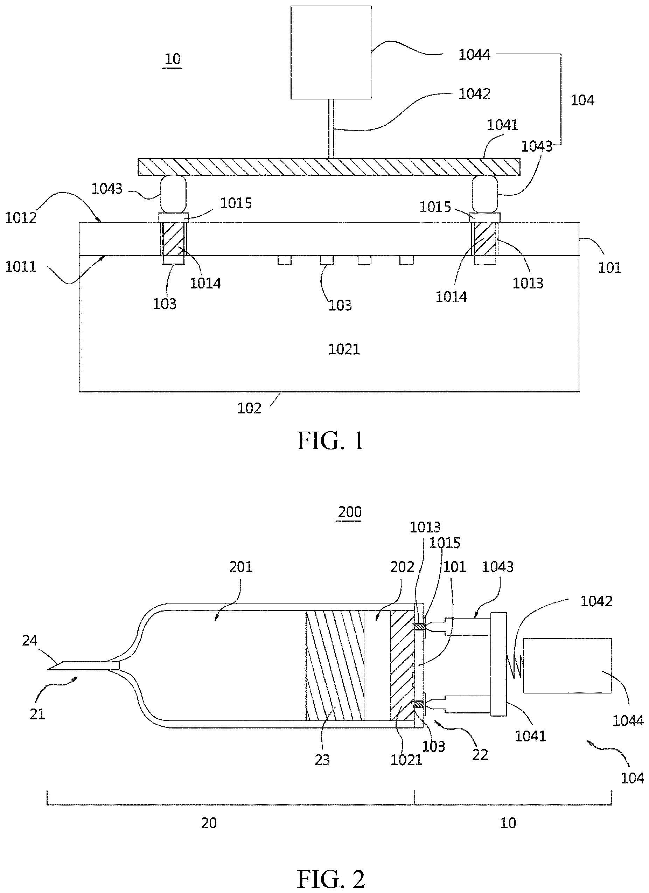

As shown in Fig. 1, provided is an electrochemical pump 10, the electrochemical pump 10 comprising: a substrate 101; a space 102 for accommodating electrolyte; a plurality of electrodes 103; and an electronic device 104.

The substrate 101 has a first surface 1011, a second surface 1012, a plurality of through holes 1013, a plurality of conductive materials 1014 and a plurality of contacts 1015. The contacts 1015 are disposed on one side of the through holes 1013, which is on the second surface 1012, and the conductive materials 1014 is disposed in the through holes 1013, the components on the first surface 1011 can thus be electrically connected the components on the second surface 1012. The space 102 for accommodating electrolyte is disposed on the first surface 1011 and contains an electrolyte 1021 therein. The electrodes 103 disposed on the first surface 1011, are in direct contact with the electrolyte 1021, and are electrically connected to the conductive materials 1014 in the through holes 1013. The electronic device 104 is disposed on the second surface 1012 and is electrically connected to the contacts 1015.

According to the invention, the two sides of the through holes 1013 can be electrically connected by filling conductive materials in the through holes 1013, coating conductive material layer on the wall of the through holes 1013, or inserting wires, but the technical means are not limited thereto. According to the invention, the feedthrough technology may be used to manufacture the through holes 1013 and the conductive materials 1014 therein, which is widely applied for manufacturing complementary metal oxide semiconductor (CMOS) processes and integrated circuit (IC) packaging, thereby achieves completely separated "wet" structure (i.e., various structures on the first surface and in direct contact with the electrolyte) and "dry" structure (i.e., various structures on the second surface that are in contact with the surface, such as the electronic device) to avoid direct contact between the electrolyte and the electronic device, and thus enhance the reliability of the overall device.

In one embodiment, the contacts 1015 are a plurality of contact pads.

According to the invention, the space 102 for accommodating electrolyte is formed by attaching a superabsorbent material to the first surface, or by attaching a breathable film to the first surface. In one embodiment, the space 102 for accommodating electrolyte is a superabsorbent material coated on the first surface 1011, and the electrolyte 1021 is formulated as gel to keep the electrodes 103 and the electrolyte 1021 in contact, to prevent the electrolyte from leaking, and to allow the generated gas to leave the space 102 for accommodating electrolyte; the electrolyte 1021 is a solution that can generate gas through the electrode, such as water or electrolytic solution, but it is not limited thereto.

In a preferred embodiment, the superabsorbent material is a sponge. In another embodiment, the superabsorbent material is added to the surfaces of the electrodes 103, and the superabsorbent material is made of a material selected from the group consisting of: polyvinyl alcohol (PVA), polyethylene oxide (PEO), polyacrylic acid, polylactic acid (PLA), polyglycolic acid (PGA), PLA/PGA copolymer and polycaprolactone (PCL).

In a preferred embodiment, the number of electrodes 103 is at least three. More specifically, two of the electrodes 103 respectively serve as an anode and a cathode. In order to have better electrochemical performance, the anode and cathode are arranged in an interdigitated comb-shaped electrode structure, and the other electrode is a reference electrode to avoid voltage degradation due to, for example, the ohmic voltage produced by the current flowing in dilute acid solution, salt water and other ion electrolytes. The voltage degradation may affect the accuracy of drug delivery and needs to be avoided to obtain accurate voltage level measurements. If four or more electrodes are provided, the remaining electrodes are redundant electrodes. These redundant electrodes can avoid the inconvenience of repairing a suddenly failed electrode.

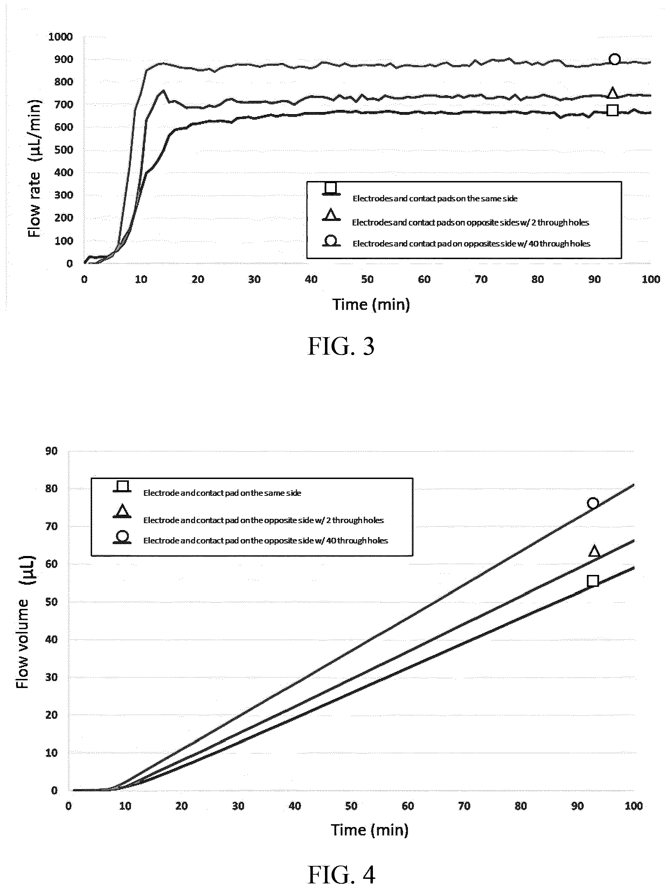

In order to improve electrochemical efficiency, any of the electrodes 103 can be electrically connected to the electronic device 104 through a plurality of through holes 1013. In a preferred embodiment, the anode and the cathode each have 20 through holes. Figs. 3 and 4 show the comparison of the electrochemical efficiency of electrodes with other designs. The design with 20 through holes in the anode and cathode (the electrodes and the contact pads are on the opposite sides, with 40 through holes) increases the flow rate and total delivery volume by about 30% compared to the design of electrodes without through holes (the electrodes and the contact pads are on the same side), and also increases the flow rate and total delivery volume by approximately 20% compared to a design of the cathode and anode respectively only has one through hole (the electrode and the contact pad are on the opposite sides, with 2 through holes).

The first surface 1011 and the electrodes 103 can be coated with a hydrophilic layer to improve electrochemical efficiency. The coating materials include but are not limited to: polyvinylphenol (PVP), polyacrylic acid (PAA), poly(vinyl phenol), polyacrylic acid (PAA), ethylene oxide (PEO), polysaccharides, proton exchange membranes (such as sulfonated tetrafluoroethylene (also known as Nafion)), nanostructured metals, epoxy resins, and a polymer fiber product made of the aforementioned materials.

The coated hydrophilic layer is used to ensure that the first surface 1011 of the electrochemical pump 10 is hydrophilic and can continuously generate gas during electrochemical reactions. The coated electrodes can provide higher gas solubility compared to conventional unprocessed electrodes.

To further enhance this electrochemical efficiency, it is effective to change the shape of the electrodes. In some embodiments, inverted trapezoidal electrodes are formed by modified photolithography procedures or modified standard electron beam lithography, modified reactive ion etching (RIE), modified deep reactive ion etching (DRIE) or modified inductively coupled plasma (ICP) with modified oxygen plasma processing. On the other hand, the electrochemical efficiency can be enhanced by changing the shape of the electrode. In one embodiment, the electrodes 103 are made into an inverted trapezoid shape to generate a strong electric field and result in a larger amount of gas generated. In another embodiment, the hydrophilicity of the electrochemical pump 10 can be achieved by hydrophilic process, such as oxygen plasma process, chemical etching and mechanical friction.

The electronic device 104 is electrically connected to the contacts 1015 and is used to provide power to the electrodes 103 and control the time and power of the electrochemical reaction. In a preferred embodiment, the electronic device 104 is detachable and can be assembled with and separated from other components of the electrochemical pump 10.

In one embodiment, the electronic device 104 includes: a circuit board 1041; an input port 1042; and an outlet 1043.

The outlet 1043 electrically connects the circuit board 1041 to the contact 1015 to control the electrodes 103, so as to control the electrochemical pump 10 to generate gas. One end of the input port 1042 is electrically connected to the circuit board 1041, and the other end is a power input port that can be electrically connected to a power source to supply power to the electrochemical pump 10. The power supply may be an external power supply or included in the electronic device 104.

In one embodiment, the electronic device 104 further includes a power supply 1044 electrically connected to the input port 1042.

In a preferred embodiment, the electronic device 104 further comprises a driving circuit component to control the switch of the electronic device 104.

Accordingly, the outlet 1043 serves as an electrical bridge between the electronic devices 104 and other components of the electrochemical pump 10. The outlet 1043 can be a micro connector, such as a pogo pin.

As shown in Fig. 2, provided is a drug delivery device 200 comprising: a container, which is a syringe 20; and the electrochemical pump 10.

The syringe 20 has a first opening 21, a second opening 22, and a spacing member 23. The first opening 21 and the second opening 22 are opposite to each other.

In a preferred embodiment, the syringe 20 further includes an injection needle 24 disposed at the first opening 21.

In one embodiment, the drug delivery device 100 is small enough to deliver a trace amount of drug, and the volume of the syringe 10 is preferably in microliter unit (0.1-500 microliter).

In another embodiment, the drug delivery device 100 can deliver high-concentration, large-volume drugs, wherein the volume of the syringe 20 is preferably in the milliliter (mL) unit (0.1-500 mL).

According to one embodiment of the present invention, the spacing member 23 is disposed inside the syringe 20 and divides the syringe 20 into a first chamber 201 and a second chamber 202. The first chamber 201 is used to accommodate the drug, and the second chamber 202 is used to accommodate the space 102 for accommodating electrolyte of the electrochemical pump and the electrolyte 1021 therein.

According to one embodiment of the present invention, the container may be a syringe 20 made of transparent or translucent material, and the container, such as a syringe, may be rigid or flexible. Preferably, the syringe 20 is made of rigid material, including but not limited to: glass (such as quartz, fused silica, alkali lime, silicate and borosilicate), polymer/plastic (such as Polycarbonate (PC), polymethyl methacrylate (PMMA), polypropylene (PP), polyethylene (PE), ethylene terephthalate (PET), polylactic acid (PLA), thermoplastic elastomer (TPE) and parylene, COP or COC cyclic olefin polymers), rubber (natural rubber and rubber), colloids (epoxy resin, silicone and acrylic resin) and conductive polymers (polyfluorene, polybiphenyl compounds , polypyrene, polyazulene, polynaphthalene, polypyrrole (PPY), polyaniline (PANI), polythiophene (PT), poly(3,4-ethylenedioxythiophene) (PEDOT), polyparaphenylene sulfide (PPS) ), polyacetylene (PAC) and polyphenylene vinylene (PPV)).

In the present embodiment, the spacing member 23 is a diaphragm. The may be made of thermoplastic elastomer (TPE), styrene-ethylene-butylene-styrene block copolymer (SEBS), styrene-ethylene-propylene-styrene block copolymer (SEPS), and polyparaethylene, but the application is not limited thereto. However, in another embodiment, the spacing member 23 can also be a blocking member, which is composed of a rubber plug which can be made of rubber, polytetrafluoroethylene (Teflon), silicone, thermoplastic elastomer ( TPE), styrene-ethylene-butylene-styrene block copolymer (SEBS), styrene-ethylene-propylene-styrene block copolymer (SEPS), and parylene.

According to the invention, the electrochemical pump 10 is disposed at the second opening 22 of the syringe 20, and the electrochemical pump 10 and the syringe 20 are sealably coupled and sealing the second opening 22 to prevent the gas generated by the electrochemical pump 10 as well as the electrolyte 1021 contained therein from leaking. As such, if the electrolyte 1021 is subjected to an electrochemical reaction and generates gas, the pressure of the second chamber 202 will be higher than the pressure of the first chamber 201, and causing the spacing member 23 to move towards the first opening 21, so as to deliver the drug. The spacing member 23 therefore must be provided to isolate the first chamber 201 and the second chamber 202 from each other to prevent the electrolyte and the drug from mixing. The sealing method includes, but is not limited to, using gaskets (such as O-rings), using adhesive seals, or using welding (such as ultrasonic, thermal or laser welding).

In one embodiment, the electrochemical pump 10 is detachable after the second opening 22 is sealed.

In another embodiment, the electrochemical pump 10 is non-detachable after the second opening 22 is sealed.

It would be obvious to those skilled in the art that the embodiments disclosed herein are illustrative, rather than limiting. Those skilled in the art can embody the invention through numerous changes and substitutions without departing from the technical features of the invention. According to the embodiments described herein, the present invention can be modified in various ways without hindering its implementation. The claims provided define the scope of the invention, which covers the aforementioned methods and structures and their equivalents.

## Claims

1. An electrochemical pump, comprising:
a substrate having a first surface, a second surface and a plurality of through holes, wherein
the second surface has a plurality of contacts, and the through holes have a conductive material therein which is electrically connected to the plurality of contacts;
a plurality of electrodes disposed on the first surface and electrically connected to the plurality of contacts on the second surface through the conductive material in the plurality of through holes;
an electronic device disposed on the second surface and electrically connected to the contacts on the second surface; and
a space for accommodating electrolyte, which has an electrolyte therein, and is adjacent to the first surface such that the plurality of electrodes is in contact with the electrolyte.

2. The electrochemical pump of claim 1, wherein the electrode at least includes an anode and a cathode.

3. The electrochemical pump of claim 2, wherein the electrode further includes a reference electrode.

4. The electrochemical pump of claim 2, wherein the electrode further includes a redundant electrode.

5. The electrochemical pump according to claim 1, wherein any of the electrodes is electrically connected to a plurality of contacts through the plurality of through holes.

6. The electrochemical pump of claim 1, wherein the first surface is coated with a hydrophilic layer.

7. The electrochemical pump of claim 1, wherein the space for accommodating electrolyte is formed by attaching a superabsorbent material to the first surface.

8. The electrochemical pump of claim 7, wherein the superabsorbent material is a sponge.

9. The electrochemical pump of claim 7, wherein the superabsorbent material is made of a material selected from the group consisting of: polyvinyl alcohol (PVA), polyethylene oxide (PEO), polyacrylic acid, poly lactic acid (PLA), polyglycolic acid (PGA), PLA/PGA copolymer, polycaprolactone (PCL), and a polymer fiber product thereof.

10. The electrochemical pump of claim 1, wherein the space for accommodating electrolyte is formed by attaching a breathable film to the first surface, wherein the space for accommodating electrolyte is sealed with the breathable film.

11. The electrochemical pump of claim 1, wherein the electronic device includes: a circuit board; an input port; and an outlet.

12. The electrochemical pump of claim 11, wherein the electronic device further includes a driving circuit component.

13. The electrochemical pump of claim 12, wherein the electronic device includes a power supply.

14. The electrochemical pump of claim 13, wherein the circuit board is electrically connected to the contact by the outlet, the power supply is electrically connected to the circuit board via the input port, and the driving circuit component is electrically connected to the circuit board and is electrically connected to the power supply.

15. The electrochemical pump of claim 1, wherein the electronic device is detachable.

16. A drug delivery device, comprising:
a container for accommodating a drug, having opposing first and second openings, wherein the first opening is an outlet of the drug;
a spacing member disposed in the container between the two openings, and capable of sliding freely along a wall of the container; and
the electrochemical pump of claim 1, disposed at the second opening, wherein the electrochemical pump is sealably attached to the container to seal the second opening.

17. The device of claim 16, wherein the container is a syringe.

18. The device of claim 17, wherein the syringe includes a needle disposed at the first opening.

19. The device of claim 16, wherein the container is transparent or translucent.

20. The device of claim 16, wherein the container is rigid or flexible.

21. The device of claim 16, wherein the spacing member is composed of a rubber plug.
